Europäisches Patentamt

European Patent Office

Office européen des brevets

(11) Veröffentlichungsnummer : **0 014 308**
**B1**

(12) **EUROPÄISCHE PATENTSCHRIFT**

(45) Veröffentlichungstag der Patentschrift :
26.01.83

(51) Int. Cl.³ : **C 07 C 69/34, C 07 C 67/08**

(21) Anmeldenummer : **80100031.6**

(22) Anmeldetag : **04.01.80**

(54) **Biologisch abbaubare, oxidationsstabile, flüssige Estergemische mit niedrigen Trübungspunkten und deren Herstellung.**

(30) Priorität : **03.02.79 DE 2904164**
**29.09.79 DE 2939663**

(43) Veröffentlichungstag der Anmeldung :
**20.08.80 Patentblatt 80/17**

(45) Bekanntmachung des Hinweises auf die Patenterteilung : **26.01.83 Patentblatt 83/04**

(84) Benannte Vertragsstaaten :
**BE DE FR GB IT NL**

(56) Entgegenhaltungen :
**FR A 2 014 645**
**NL A 123 948**
**US A 3 119 849**
**US A 3 776 928**

(73) Patentinhaber : **DYNAMIT NOBEL AKTIENGESELL-SCHAFT**
**Patentabteilung Postfach 1209**
**D-5210 Troisdorf, Bez. Köln (DE)**

(72) Erfinder : **Naskar, Sasanka Sekhar**
**Parkweg 63**
**D-5810 Witten (DE)**
Erfinder : **Pass, Reinhard**
**Brink 10**
**D-5810 Witten (DE)**

EP 0 014 308 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**0 014 308**

Biologisch abbaubare, oxidationsstabile, flüssige Estergemische mit niedrigen Trübungspunkten und deren Herstellung

Die Erfindung betrifft biologisch vollständig abbaubare, toxikologisch unbedenkliche, oxidationsstabile, geschmacks- und geruchsneutrale, helle, flüssige, einheitliche Estergemische mit niedrigen Trübungspunkten, niedrigen Verdampfungsverlusten bei erhöhten Temperaturen, beliebig einstellbaren Viskositäten bzw. Dichten, bestehend aus Veresterungsprodukten von 1 Mol Glycerin mit 1,4 bis 2,8 Mol, vorzugsweise 1,6 bis 2,6 Mol, einer gesättigten geradkettigen Monocarbonsäure mit 6 bis 10 C-Atomen oder deren Gemischen und 0,1 bis 0,8 Mol, vorzugsweise 0,2 bis 0,7 Mol einer gesättigten aliphatischen Dicarbonsäure, deren Gemischen oder Anhydriden. Die neuen Estergemische besitzen Hydroxylzahlen von 5 bis 20 und eine Säurezahl unter 5.

Ziel der Erfindung ist es, flüssige einheitliche Estergemische, deren physikalische Eigenschaften in bezug auf Stock- bzw. Trübungspunkte und Oxidationsstabilität denen der natürlich vorkommenden Öle überlegen und deren Viskosität und Dichte je nach Verwendungszweck und Anwendungserfordernissen einstellbar sind, aus technisch zugänglichen Rohstoffen nach einem einfachen, wirtschaftlich wenig aufwendigen Verfahren zu gewinnen. Insbesondere bezieht sich die Erfindung auf Stoffsysteme, deren vollständige biologische Abbaubarkeit und somit die umweltfreundliche Unbedenklichkeit gewährleistet ist. Die erfindungsgemäß hergestellten Estergemische finden besondere Anwendung im kosmetischen und technischen Sektor bei der Metallumformung, Glasfaserbehandlung und Schmierstoffzusätzen.

Öle aus natürlichen Vorkommen, welche bei Raumtemperatur flüssig vorliegen, bieten wegen ihrer vollständigen biologischen Abbaubarkeit Verwendungsmöglichkeiten im kosmetischen und technischen Sektor. Die Anforderungen der verschiedenen Industriezweige bezüglich hoher Qualität und spezifischen Eigenschaften für bestimmte Anwendungswecke sind so differenziert, daß ein Öl aus natürlichen Vorkommen diese technischen Voraussetzungen nicht immer erfüllen kann.

Die Trübungspunkte bzw. Kältestabilität der natürlichen Öle liegt kaum unter 8 °C. Die Viskositäten und Dichten sind zu eng begrenzt, so z.B. schwankt die Viskosität bei 20 °C zwischen 50 und 90 mPs (Ausnahme Ricinusöl mit ca. 1 000 mPs) und die Dichte bei 25 °C um 0,9.

Eine zusätzliche negative Eigenschaft natürlicher Öle ist ihre schlechte Oxidationsstabilität ; sie neigen demzufolge zum Verharzen und scheiden u.a. aggressive Fettsäuren aus. Dieses Verhalten ist auf den Gehalt an ungesättigten Fettsäuren zurückführen. Versuche, einige dieser Nachteile — insbesondere fehlende Oxidationsstabilität — zu beheben, führten zu Triglyceriden mittelkettig gesättigter Fettsäuren, die geschmacks- und geruchsneutral, oxidationsstabil, biologisch abbaubare flüssige Estergemische sind. Die Viskosität solcher Produkte Miglyol 812 [R] der Firma Dynamit Nobel AG beträgt bei 20 °C ca. 30 mPs und der Trübungspunkt unterschreitet kaum − 5 °C.

Möglichkeiten, die Nachteile der natürlichen Öle auszugleichen, beitet die Anwendung von Paraffinölen. Gereinigte Paraffinöle, sogenannte Weißöle, klare farblose Flüssigkeiten von öliger Beschaffenheit, sind Mischungen gesättigter Kohlenwasserstoffe mit verschiedenen Viskositäten und niedrigen Trübungspunkten. Sie sind oxidationsstabil und finden eine breite Anwendung im kosmetischen und technischen Sektor. Ein großer Nachteil aber ist ihre geringe biologische Abbaubarkeit und hohe Verdampfungsverluste bei erhöhten Temperaturen.

Die Aufgabe der Erfindung war daher die Entwicklung und wirtschaftliche Herstellung eines biologisch vollständig abbaubaren, oxidationsstabilen, flüssiger einheitlichen Estergemisches mit niedrigen Stock- bzw. Trübungspunkten und guten Viskositätsindices, welches relativ niedrige Verdampfungsverluste bei erhöhten Temperaturen aufweist und besonders Anwendung im kosmetischen und technischen Sektor wegen seiner differenzierten physikalischen Eigenschaften findet, sowie die Verwendung von weiteren aliphatischen Dicarbonsäuren anstelle von Bernsteinsäure oder deren Anhydrid.

Gegenstand der Erfindung sind flüssige Glyceridgemische nach Anspruch 1 mit Gehalten von 1,4 bis 2,8 Mol gesättigter, geradkettiger Monocarbonsäureresten der Kettenlänge von 6 bis 10 C-Atomen und 0,1 bis 0,8 Mol gesättigten aliphatischen Dicarbonsäureresten, worin im jeweiligen Produkt die Summe der Säurereste stets 3 Mol je Mol Glycerin beträgt, so daß die Produkte praktisch frei von Hydroxylgruppen sind. Überraschend werden bei steigenden Gehalten von 0,1 bis 0,8 Mol aliphatischen Dicarbonsäuren und entsprechend abnehmenden Gehalten von 2,8 bis 1,4 Mol der gesättigten geradkettigen Monocarbonsäuren Veresterungsprodukte mit sehr niedrigen Trübungspunkten, jedoch ansteigenden Viskositätsindices erhalten. Hierbei wird mit steigendem Gehalt der aliphatischen Dicarbonsäuren ein Absinken der Trübungspunkte zu sehr niedrigen Temperaturen und entsprechend ein Anstieg der Viskosität zu sehr hohen Zähigkeiten erreicht. Solche Estergemische sind biologisch vollständig abbaubare, oxidationsstabile, geschmacks- und geruchsneutrale, helle, flüssige einheitliche Estergemische mit niedrigen Stock- bzw. Trübungspunkten, niedrigen Verdampfungsverlusten bei erhöhten Temperaturen und guten bzw. beliebig einstellbaren Viskositätsindices bzw. Dichten.

Als aliphatische Dicarbonsäuren sollen die gesättigten Dicarbonsäuren, besonders mit 4 bis 10 C-Atomen verstanden werden. Insbesondere kommen die $\alpha$, $\omega$-Dicarbonsäuren infrage, jedoch sollen gegebenenfalls Dicarbonsäuren mit verzweigter Kette wie die Methylglutarsäure nicht gänzlich ausgeschlossen sein. Bevorzugte Säuren sind Bernstein-, Glutar-, Adipin-, Sebacin- und Azelainsäure. Mischungen der Dicarbonsäuren sowie ggf. die Anhydride sind verwendbar.

Als gesättigte Monocarbonsäuren sollen die geradkettigen Fettsäuren verstanden werden.

0 014 308

Die Nachteile der natürlich vorkommenden Öle, wie unangenehmer Geruch, schlechte Oxidationsstabilität, höhere Trübungspunkte, niedrige Viskosität bzw. Dichte bzw. die geringe bilogische Abbaubarkeit der Paraffinöle und hohe Verdampfungsverluste bei erhöhten Temperaturen, treten bei den Stoffen nach der Erfindung nicht auf. Die Produkte besitzen im allgemeinen, in Abhängigkeit vom jeweiligen Gehalt an Dicarbonsäureestern im Veresterungsprodukt, Trübungspunkte von − 16 bis − 70 °C, Viskositäten von 50 bis 5 000 mPas, Dichten von 0,96 bis 1,07 und Verdampfungsverluste von 14 bis 32 % während 100 Stunden bei 150 °C bei Normaldruck.

Weiterer Gegenstand der Erfindung ist ein Verfahren zur Herstellung solcher Estergemische, dadurch, daß man je Mol Glycerin mit 1,4-2,8 Mol, vorzugsweise 1,6-2,6 Mol einer gesättigten, geradkettigen Monocarbonsäure mit 6-10 C-Atomen oder deren Gemischen und 0,1 bis 0,8 Mol, vorzugsweise 0,2 bis 0,6 Mol, gesättigten, aliphatischen Dicarbonsäuren, deren Gemischen oder Anhydriden bei 200-250 °C, vorzugsweise unter Vakuum, bei Molverhältnissen vom Glycerin zu Dicarbonsäuren unter 1 : 0,6 gemeinsam verestert oder nach Bildung hydroxylhaltiger Partialester der Monocarbonsäure, bis Säurezahlen unter 50, vorzugsweise unter 10, erreicht sind, diese mit 0,1 bis 0,8 Mol Dicarbonsäure verestert, bis eine Hydroxylzahl von 5-20 und eine Säurezahl unter 5, vorzugsweise unter 1, erreicht ist, und daß man das Rohprodukt in üblicher Weise entfärbt und desodorisiert.

Die Herstellung wird bevorzugt nach den Ansprüchen 3 bis 5 ausgeführt.

Soweit zuerst Partialester der Monocarbonsäuren hergestellt werden, sollen diese Säurezahlen unter 50, vorzugsweise unter 10, erreichen.

Bei Molverhältnissen von Glycerin zu Dicarbonsäuren unter 1 : 0,6 kann man alle Reaktionsteilnehmer gleichzeitig verestern. Bei höheren Molverhältnissen erfolgt die Zugabe von Dicarbonsäuren erst nach der Abkühlung der Fettsäurepartialester auf 140 °C. In beiden Verfahren, ob die Zugabe der aliphatischen Dicarbonsäuren gleichzeitig oder anschließend erfolgt, ist die Veresterung unter Vakuum dann vorzunehmen, wenn die Säurezahl während der Veresterung bei 220-240 °C unter 60 liegt. Nach Erreichen von 350 mbar wird das Vakuum anschließend um 5-50 mbar pro Stunde verbessert, bis die Säurezahl unter 5, vorzugsweise unter 1, sinkt. Hierbei ist die Verwendung eines Dephlegmators sehr nützlich.

Die Produkte sind physiologisch unbedenklich.

Die bei der Herstellung der Veresterungsprodukte verwendete Cocosvorlauffettsäure stellt einen Destillationsschnitt mit ca. 1 % $C_6$-, 58 % $C_8$- und 41 % $C_{10}$-Säure dar, der während der Destillation der Cocosfettsäure anfällt.

Die Verwendung der reinen $C_6$-$C_{10}$-Fettsäuren ist ebenfalls möglich, doch ergeben sich keine besonderen Vorteile der Eigenschaften gegenüber Fettsäuregemischen. Natürliche Heptansäure ist auch im Handel erhältlich (ATO-Chemie).

Die erfindungsgemäß erhaltenen Veresterungsprodukte auf Basis Glycerin, gesättigten aliphatischen Monocarbonsäuregemischen mit 6 bis 10 C-Atomen bzw. Heptansäure und gesättigten aliphatischen Dicarbonsäuren oder deren Gemischen zeichnen sich durch folgende besondere Vorteile aus :

1. Es sind geruchslose, geschmacksneutrale und helle einheitliche Flüssigkeiten mit niedrigen Trübungspunkten von − 16 bis − 70 °C. Die Trübungspunkte von natürlichen Ölen liegen viel höher, z.B. von Erdnußöl ca. 8 °C. Miglyol 812 [R], ein Triglycerid der $C_8$-$C_{10}$-Fettsäuren, hat einen Trübungspunkt von − 5 °C.

2. Oxidationsstabilität gegen Luftsauerstoff bei Lagerung ist ausgezeichnet wegen Fehlens der ungesättigten Verbindungen. Alle natürlichen Öle enthalten dagegen ungesättigte Fettsäuren, welche Ranzidität und unangenehmen Geruch bei Lagerung verursachen.

3. Die Viskosität bzw. Dichte kann man beliebig, je nach Einbau der aliphatischen Dicarbonsäuren in den Veresterungsprodukten, einstellen. Die Viskosität der natürlichen Öle variiert zwischen 60-90 mPs bei 20 °C ; die Dichte beträgt ca. 0,9 bei 25 °C. Dagegen beträgt die Viskosität der entwickelten Produkte ca. 50-3 200 mm²/sec und die Dichte ca. 0,966-1,078 bei 20 °C.

4. Die Verdampfungsverluste bei erhöhten Temperaturen werden durch Einbau der Dicarbonsäure in die Veresterungsprodukte geringer. Miglyol 812 [R] weist einen Verlust von 42,9 % bei 150 °C während 100 Stunden auf. Durch steigende Menge aliphatischer Dicarbonsäuren werden die Flüchtigkeiten deutlich geringer. Sogar ein Paraffinöl mit vergleichbarer Viskosität hat einen deutlich höheren Verdampfungsverlust, z.B. hat das dickflüssige Paraffinöl (202 mm²/sec) einen Verlust von 39,3 %, ein Adipinsäure-veresterter $C_6$-$C_{10}$-Glycerinpartialester mit gleicher Viskosität dagegen 21,8 %.

5. Die Veresterungsprodukte sind wie die natürlichen Öle biologisch vollständig abbaubar, dagegen sind die Paraffinöle biologisch kaum abbaubar.

6. Die erfindungsgemäßen Estergemische weisen gegenüber herkömmlichen Mineralölprodukten und natürlich vorkommenden Ölen gleicher Viskosität neben niedrigeren Stockpunkten sehr gute Viskositätsindices auf. Die Viskositäten derartiger Estergemische liegen bei 40 °C um ca. 20-1 000 mPas,* vorzugsweise 30-150 mPas und bei 100 °C um 5-100 mPas, vorzugsweise 10-30 mPas. Die Stockpunkte liegen im allgemeinen unter − 30 °C.

Die nachfolgenden Beispiele erläutern die Erfindung näher, ohne diese jedoch zu beschränken :

* = milli pascal

3

# 0 014 308

## Beispiel 1

In einem 2-Liter-Dreihalskolben, der mit Rührwerk, Wasserabscheider, Dephlegmator, Thermometer und Gaseinleitungsrohr versehen ist, wird eine Mischung von 276 g (3 Mol) Glycerin, 88 g (0,6 Mol) Adipinsäure und 1 209 g (7,8 Mol) Cocosvorlauffettsäure ($C_6$ bis $C_{10}$-Fettsäuregemisch) in Gegenwart von 0,5 g Isopropyltitanat unter Rühren und gleichzeitigem Durchleiten von Inertgas 2 Stunden bei 240 °C und 350 mbar erhitzt und das Reaktionswasser bis zu einer Säurezahl von 60 entfernt. Bei schrittweiser Erhöhung des Vakuums (50 mbar/Stunde) bei 240 °C Reaktionstemperatur wird bis zu einer Säurezahl kleiner als 2 weiterverestert, wobei das Reaktionswasser und das entstehende Zwischenprodukt mittels eines auf 100-120 °C temperierten Dephlegmators derart getrennt werden, daß lediglich das Reaktionswasser entfernt wird. Mit abnehmender Säurezahl wird hierbei das Zwischenprodukt vollständig umgesetzt. Der erhaltene Rohester wird 2 Stunden bei 170 °C und 10 mbar desodorisiert und unter Zusatz von 10 g Bleicherde und 5 g Filterhilfsmittel 0,5 Stunden bei 120 °C gebleicht und anschließend druckfiltriert.

Säurezahl = 0,4 ; Hydroxylzahl = 8,0 ;
Viskositäten 20 °C = 54,9 mm²/sec, 37,8 °C = 23,6 mm²/sec, 98,8 °C = 5,0 mm²/sec.
Dichte 20 °C = 0,966
Trübungspunkt = − 22 °C
Verdampfungsverlust 100 Stunden/100 °C = 31,3 %
Stockpunkt = − 26 °C
Viskositätsindex = 144

## Beispiel 2

Unter Beibehaltung der im Beispiel 1 beschriebenen Reaktionsapparatur wird eine Mischung von 322 g (3,5 Mol) Glycerin, 205 g (1,4 Mol) Adipinsäure und 1 209 g (7,8 Mol) Cocosvorlauffettsäure in Gegenwart von 0,5 g Butyltitanat als Katalysator unter Inertgas 3 Stunden bei 240 °C und 350 mbar bis zu einer Säurezahl von 40 verestert. Bei schrittweiser Verbesserung des Vakuums (50 mbar/Stunde) und Entfernung des restlichen Reaktionswassers nach der in Beispiel 1 beschriebenen Art wird bei 240 °C bis zu einer Säurezahl unter 1 weiterverestert. Der erhaltene Rohester wird wie in Beispiel 1 desodorisiert, gebleicht und filtriert.

Säurezahl = 0,8 ; Hydroxylzahl = 11,5
Dichte 20 °C = 0,987
Viskositäten 20 °C = 119 mm²/sec., 37,8 °C = 45,8 mm²/sec., 98,8 °C = 8,1 mm²/sec.
Trübungspunkt = − 38 °C
Verdampfungsverlust 100 Stunden/100 °C = 23,4 %
Stockpunkt = − 44 °C
Viskositätsindex = 149

## Beispiel 3

In einem 10 Liter-Dreihalskolben, versehen mit Rührer, Wasserabscheider, Dephlegmator, Thermometer und Inertgas-Einleitungsrohr wird eine Mischung auf 1 630 g (17,7 Mol) Glycerin, 1 374 g (9,4 Mol) Adipinsäure und 5 317 g (34,3 Mol) Cocosvorlauffettsäure in Gegenwart von 3 g Butyltitanat und unter Inertgas bei 240 °C und 350 mbar bis zu einer Säurezahl von 65 verestert. Innerhalb von 6 Stunden wird das Vakuum um ca. 50 mbar/Stunde bei 240 °C verbessert. Das hierbei anfallende Reaktionswasser wird mittels eines auf 100-120 °C temperierten Dephlegmators vom Zwischenprodukt getrennt und bis zu einer Säurezahl kleiner als 1 weiterverestert.

Der Rohester wird 4 Stunden bei 170 °C und 10 mbar desodorisiert, unter Zusatz von 40 g Bleicherde und 20 g Filterhilfsmittel 1 Stunde bei 120 °C gebleicht und anschließend druckfiltriert.

Säurezahl = 0,6 ; Hydroxylzahl = 9,2
Dichte 20 °C = 1,007
Viskositäten 20 °C = 228 mm²/sec., 37,8 °C = 78,0 mm²/sec., 98,8 °C = 12,5 mm²/sec.
Trübungspunkt = − 55 °C
Verdampfungsverlust 100 Stunden/100 °C = 22,3 %
Stockpunkt = − 48 °C
Viskositätsindex = 150

## Beispiel 4

Analog Beispiel 3 werden 1 501 g (16,3 Mol) Glycerin, 1 432 g (9,8 Mol) Adipinsäure und 4 542 g (29,3 Mol) Cocosvorlauffettsäure in Gegenwart von 3 g 2-Äthylhexyltitanat bis zu einer Säurezahl kleiner als 2 verestert, desodorisiert, gebleicht und filtriert.

Säurezahl = 0,6 ; Hydroxylzahl = 12,9
Dichte 20 °C = 1,020
Viskositäten 20 °C = 358 mm²/sec., 37,8 °C = 119 mm²/sec., 98,8 °C = 16,7 mm²/sec.

4

Trübungspunkt = − 64 °C
Verdampfungsverlust 100 Stunden/100 °C = 20,6 %
Stockpunkt = − 41 °C
Viskositätsindex = 151

## Beispiel 5

In einem 2 Liter-Dreihalskolben, der mit Rührwerk, Dephlegmator, Wasserabscheider, Thermometer und Inertgaseinleitungsrohr versehen ist, wird eine Mischung aus 396 g (4,3 Mol) Glycerin und 930 g (6 Mol) Cocosvorlauffettsäure in Gegenwart von 0,5 g Isopropyltitanat bei 240 °C und 350 mbar unter Rühren und gleichzeitigem Durchleiten von Inertgas erhitzt und das Reaktionswasser bis zu einer Säurezahl kleiner als 10 entfernt. Der erhaltene Partialester wird auf 140 °C abgekühlt. Nach Zugabe von 504 g (3,45 Mol) Adipinsäure wird unter Rühren und Inertgas auf 240 °C aufgeheizt und das anfallende Reaktionswasser wird mittels eines auf 100-120 °C temperierten Dephlegmators derart getrennt, daß lediglich das Reaktionswasser entfernt und das Vakuum schrittweise bis zu 5 mbar verbessert wird. Nach Erreichen einer Säurezahl kleiner als 1 wird der Rohester abgekühlt und üblicherweise desodorisiert, gebleicht und filtriert.

Säurezahl = 0,7 ; Hydroxylzahl = 16,5
Dichte 20 °C = 1,050
Viskositäten 20 °C = 2410 mm²/sec., 37,8 °C = 670 mm²/sec., 98,8 °C = 68,3 mm²/sec.
Trübungspunkt = unter − 70 °C
Verdampfungsverlust 100 Stunden/100 °C = 16,9 %      Stockpunkt = − 27 °C
Viskositätsindex = 177

## Beispiel 6

Analog Beispiel 1 werden 276 g (3 Mol) Glycerin, 88 g (0,6 Mol) Adipinsäure und 1 016 g (7,8 Mol) Heptansäure verestert.

Säurezahl = 0,6 ; Hydroxylzahl = 17,8
Dichte 20 °C = 0,992
Viskositäten 20 °C = 39,2 mm²/sec., 37,8 °C = 18,0 mm²/sec., 98,8 °C = 4,2 mm²/sec.
Trübungspunkt = unter − 70 °C
Stockpunkt = − 66 °C
Viskositätsindex = 141

## Beispiel 7

Analog Beispiel 2 werden 322 g (3,5 Mol) Glycerin, 205 g (1,4 Mol) Adipinsäure und 1 016 g (7,8 Mol) Heptansäure verestert.

Säurezahl = 0,6 ; Hydroxylzahl = 15,7
Dichte 20 °C = 1,016
Viskositäten 20 °C = 90,1 mm²/sec., 37,8 °C = 38,9 mm²/sec., 98,8 °C = 7,1 mm²/sec.
Trübungspunkt = unter − 70 °C
Stockpunkt = − 55 °C
Viskositätsindex = 147

## Beispiel 8

Analog Beispiel 3 werden 1 501 g (16,3 Mol) Glycerin, 1 432 g (9,8 Mol) Adipinsäure und 3 815 g (29,3 Mol) Heptansäure verestert.

Säurezahl = 0,8 ; Hydroxylzahl = 4,3
Dichte 20 °C = 1,041
Viskositäten 20 °C = 304 mm²/sec., 37,8 °C = 114 mm²/sec., 98,8 °C = 16,2 mm²/sec.
Trübungspunkt = unter − 70 °C
Stockpunkt = − 44 °C
Viskositätsindex = 157

## Beispiel 9

Analog Beispiel 5 werden 396 g (4,3 Mol) Glycerin, 781 g (6 Mol) Heptansäure und 504 g (3,45 Mol) Adipinsäure verestert.

Säurezahl = 0,4 ; Hydroxylzahl = 14,3
Dichte 20 °C = 1,078
Viskositäten 20 °C = 3 175 mm²/sec., 37,8 °C = 976 mm²/sec., 98,8 °C = 89,0 mm²/sec.
Trübungspunkt = unter − 70 °C

Stockpunkt = − 30 °C
Viskositätsindex = 177

## Beispiel 10

Analog Beispiel 1 werden 276 g (3 Mol) Glycerin, 121 g (0,6 Mol) Sebacinsäure und 1 209 g (7,8 Mol) Cocosvorlauffettsäure verestert.
Säurezahl = 0,4 ; Hydroxylzahl = 5,8
Dichte 20 °C = 0,962
Viskositäten 20 °C = 60,6 mm²/sec., 37,8 °C = 26,2 mm²/sec., 98,8 °C = 5,5 mm²/sec.
Trübungspunkt = − 18 °C
Verdampfungsverlust 100 Stunden/100 °C = 32,2 %
Stockpunkt = − 16 °C
Viskositätsindex = 154

## Beispiel 11

Analog Beispiel 2 werden 322 g (3,5 Mol) Glycerin, 283 g (1,4 Mol) Sebacinsäure und 1 209 g (7,8 Mol) Cocosvorlauffettsäure verestert.
Säurezahl = 1,0 ; Hydroxylzahl = 0,8
Dichte 20 °C = 0,978
Viskositäten 20 °C = 129 mm²/sec., 37,8 °C = 49,7 mm²/sec., 98,8 °C = 9,0 mm²/sec.
Trübungspunkt = − 33 °C
Verdampfungsverlust 100 Stunden/100 °C = 26 %
Stockpunkt = − 35 °C
Viskositätsindex = 164

## Beispiel 12

Analog Beispiel 4 werden 1 501 g (16,3 Mol) Glycerin, 1 982 g (9,8 Mol) Sebacinsäure und 4 542 g (29,3 Mol) Cocosvorlauffettsäure verestert.
Säurezahl = 0,4 ; Hydroxylzahl = 13,8
Dichte 20 °C = 0,996
Viskositäten 20 °C = 483 mm²/sec., 37,8 °C = 166 mm²/sec., 98,8 °C = 23,1 mm²/sec.
Trübungspunkt = − 34 °C
Verdampfungsverlust 100 Stunden/100 °C = 21,4 %
Stockpunkt = − 36 °C
Viskositätsindex = 168

## Beispiel 13

Analog Beispiel 2 werden 276 g (3,0 Mol) Glycerin, 311 g (1,65 Mol) Azelainsäure und 884 g (5,7 Mol) Cocosvorlauffettsäure verestert.
Säurezahl = 0,4 ; Hydroxylzahl = 6,5
Dichte 20 °C = 0,999
Viskositäten 20 °C = 319 mm²/sec., 37,8 °C = 112 mm²/sec., 98,8 °C = 16,5 mm²/sec.
Trübungspunkt = − 46 °C
Verdampfungsverlust 100 Stunden/100 °C = 18,1 %
Stockpunkt = − 42 °C
Viskositätsindex = 162

## Beispiel 14

Analog Beispiel 2 werden 322 g (3,5 Mol) Glycerin, 40 g Glutarsäure (0,35 Mol) und 1 322 g (10,15 Mol) Heptansäure verestert.
Säurezahl = 0,18
Hydroxylzahl = 16,7
Dichte 20 °C = 0,984
Viskosität 20 °C = 38,5 mm²/sec.
Trübungspunkt = unter − 70 °C
Stockpunkt = − 66 °C

## Beispiel. 15

In einem 2 Liter-Dreihalskolben, der mit Rührwerk, Wasserabscheider, Dephlegmator, Thermometer

6

und Gaseinleitungsrohr versehen ist, wird eine Mischung von 276,3 g (3 Mol) Glycerin, 60 g (0,6 Mol) Bernsteinsäureanhydrid und 1 202 g (7,8 Mol) Cocosvorlauffettsäure ($C_6$ bis $C_{10}$-Fettsäuregemisch) in Gegenwart von 0,5 g Isopropyltitanat unter Rühren und gleichzeitigem Durchleiten von Inertgas 2 Stunden bei 240 °C und 350 mbar erhitzt und das Reaktionswasser bis zu einer Säurezahl von 60 entfernt. Bei schrittweiser Erhöhung des Vakuums (50 mbar/Stunde), bei 240 °C Reaktionstemperatur, wird bis zu einer Säurezahl kleiner als 2 weiterverestert, wobei das Reaktionswasser und das entstehende Zwischenprodukt mittels eines auf 100-120 °C temperierten Dephlegmators derart getrennt werden, daß lediglich das Reaktionswasser entfernt wird. Mit abnehmender Säurezahl wird hierbei das Zwischenprodukt vollständig umgesetzt. Der erhaltene Rohester wird 2 Stunden bei 170 °C und 10 mbar desodorisiert und unter Zusatz von 10 g Bleicherde und 5 g Filterhilfsmittel 0,5 Stunden bei 120 °C gebleicht und anschließend druckfiltriert. Säurezahl = 0,2 ; Verseifungszahl 360 ; Hydroxylzahl 8,4 ; Hazenfarbzahl 50 ; Dichte 20 °C = 0,966 ; Viskosität 20 °C = 52,4 mPas ; Trübungspunkt − 16 °C ; Verdampfungsverlust 100 Stunden/100 °C = 31,3 %.

### Beispiel 16

Unter Beibehaltung der im Beispiel 15 beschriebenen Reaktionsapparatur wird eine Mischung von 322 g (3,5 Mol) Glycerin, 140 g (1,4 Mol) Bernsteinsäureanhydrid und 1 186 g (7,8 Mol) Cocosvorlauffettsäure ($C_6$ bis $C_{10}$-Fettsäuregemisch) in Gegenwart von 0,5 g Butyltitanat als Katalysator unter Inertgas 3 Stunden bei 240 °C und 350 mbar bis zu einer Säurezahl von 40 verestert. Bei schrittweiser Verbesserung des Vakuums (50 mbar/Stunde) und Entfernung des restlichen Reaktionswassers nach der in Beispiel 15 beschriebenen Art wird bei 240 °C bis zu einer Säurezahl unter 1 weiterverestert. Der erhaltene Rohester wird wie in Beispiel 15 desodorisiert, gebleicht und filtriert. Säurezahl 0,2 ; Verseifungszahl 393 ; Hydroxylzahl 8,6 ; Hazenfarbzahl 50 ; Dichte 20 °C = 0,992 ; Viskosität 20 °C = 116,8 mPas ; Trübungspunkt − 37 °C ; Verdampfungsverlust 100 Stunden/100° = 24,9 %.

### Beispiel 17

In einen 10 Liter-Dreihalskolben, versehen mit Rührer, Wasserabscheider, Dephlegmator, Thermometer, Inertgas-Einleitungsrohr, wird eine Mischung aus 1 628 g (17,7 Mol) Glycerin, 972 g (9,7 Mol) Bernsteinsäureanhydrid und 5 173 g (33,6 Mol) Cocosvorlauffettsäure ($C_6$ bis $C_{10}$-Fettsäuregemisch) in Gegenwart von 3 g Butyltitanat und unter Inertgas bei 240 °C und 350 mbar bis zu einer Säurezahl von 65 verestert. Innerhalb von 6 Stunden wird das Vakuum um ca. 50 mbar/Stunde bei 240 °C verbessert. Das hierbei anfallende Reaktionswasser wird mittels eines auf 100-120 °C temperierten Dephlegmators vom Zwischenprodukt getrennt und bis zu einer Säurezahl kleiner als 1 weiterverestert.

Der Rohester wird 4 Stunden bei 170 °C und 10 mbar desodorisiert, unter Zusatz von 40 g Bleicherde und 20 g Filterhilfsmittel 1 Stunde bei 120 °C gebleicht und anschließend druckfiltriert. Säurezahl 0,6 ; Verseifungszahl 418 ; Hydroxylzahl 9,2 ; Hazenfarbzahl 60 ; Dichte 20 °C = 1,012 ; Viskosität 20 °C = 257 mPas ; Trübungspunkt − 51 °C ; Verdampfungsverlust 100 Stunden/100 °C = 20,3 %.

### Beispiel 18

Analog Beispiel 17 werden 1 503 g (16,3 Mol) Glycerin, 980 g (9,8 Mol) Bernsteinsäureanhydrid und 4 523 g (29,4 Mol) Cocosvorlauffettsäure in Gegenwart von 3 g 2-Äthylexyltitanat bis zu einer Säurezahl kleiner als 2 verestert, desodorisiert, gebleicht und filtriert. Säurezahl 1,0 ; Verseifungszahl 433 ; Hydroxylzahl 7,5 ; Hazenfarbzahl 60 ; Dichte 20° = 1,026 ; Viskosität 20 °C = 382 mPas ; Trübungspunkt kleiner als − 30 °C ; Verdampfungsverlust 100 Stunden/100 °C = 19,4 %.

### Beispiel 19

In einem 2 Liter-Dreihalskolben, der mit Rührwerk, Dephlegmator, Wasserabscheider, Thermometer und Inertgaseinleitungsrohr versehen ist, wird eine Mischung aus 396 g (4,3 Mol) Glycerin und 928 g (6 Mol) Cocosvorlauffettsäure in Gegenwart von 0,5 g Isopropyltitanat unter Rühren und gleichzeitigem Durchleiten von Inertgas bei 240 °C und 350 mbar erhitzt und das Reaktionswasser bis zu einer Säurezahl kleiner als 10 entfernt. Der erhaltene Partialester wird auf 140 °C abgekühlt. Nach Zugabe von 345 g (3,45 Mol) Bernsteinsäureanhydrid wird unter Rühren und Inertgas auf 240 °C aufgeheizt, und das anfallende Reaktionswasser wird mittels eines auf 100-120 °C temperierten Dephlegmators derart getrennt, daß lediglich das Reaktionswasser entfernt und das Vakuum schrittweise bis zu 5 mbar verbessert wird. Nach Erreichen einer Säurezahl kleiner als 1 wird der Rohester abgekühlt und üblicherweise desodorisiert, gebleicht und filtriert. Säurezahl 0,24 ; Verseifungszahl 478 ; Hydroxylzahl 13,6 ; Hazenfarbzahl 70 ; Dichte 20 °C = 1,069 ; Viskosität 20 °C = 4 463 mPas ; Trübungspunkt kleiner als − 70 °C ; Verdampfungsverlust 14,1 %.

Die nachfolgende Tabelle 1 beschreibt die aus den Beispielen erhaltenen entwickelten Produkte näher und vergleicht sie mit natürlich vorkommendem Öl, z.B. Erdnußöl und einem mittelviskosen Paraffinöl des Handels.

| Beispiele | Zusammensetzung | Mole B je Mole G | SZ | VZ | OHZ | Trübungs- punkt °C | Verdampfungs- verlust 150 °C/100 h | Viskosität (mm²/s) | Dichte (20 °C) |
|---|---|---|---|---|---|---|---|---|---|
| Miglyol$^R$ | ** | | 0,1 | 340 | 5,1 | 5 | 42,9 % | 29,8 | 0,950 |
| Erdnußöl | *** | | 0,11 | 188 | 3,8 | 8 | 6,8 % | 93,6 | 0,911 |
| Paraffinöl | **** | | 0,1 | 0 | 0 | − 19 | 39,3 % | 202 | 0,880 |
| Beisp. 15 | Bernsteinsäure verest. Cocosvor- lauffettsäure Partialester | 0,20 | 0,4 | 360 | 8,4 | − 16 | 31,3 % | 52,4 | 0,966 |
| Beisp. 16 | " | 0,40 | 0,3 | 393 | 8,6 | − 37 | 24,9 % | 116 | 0,992 |
| Beisp. 17 | " | 0,54 | 0,25 | 418 | 9,2 | − 51 | 20,3 % | 222 | 1,008 |
| Beisp. 18 | " | 0,60 | 0,6 | 433 | 7,5 | unter − 30 | 19,4 % | 382 | 1,026 |
| Beisp. 19 | " | 0,80 | 0,24 | 478 | 13,6 | unter − 70 | 14,1 % | 4 463 | 1,069 |

\* Miglyol 812 ® = Triglyceride gesättigter Säuren ($C_6$ 0,5-1 Gew.-% ; $C_8$ 63-64 Gew.-% ; $C_{10}$ 34-35 Gew.-% ; $C_{12}$ 0,5-2 Gew.-%)

\*\* Cocosvorlauffettsäuretriglycerid

\*\*\* Triglycerid auf Basis vorwiegend ungesättigter Fettsäure und $C_{16}$-Säure

\*\*\*\* Isoparaffin

B = Bernsteinsäure bzw. Bernsteinsäureanhydrid ; G = Glycerin ; SZ = Säurezahl ; VZ = Versei-fungszahl ; OHZ = Hydroxylzahl

## Ansprüche

1. Biologisch vollständig abbaubare, flüssige, einheitliche Estergemische mit niedrigen Trübungspunkten und für jeweilige Produkte einstellbaren Viskositäten, bestehend aus Veresterungsprodukten von 1 Mol Glycerin mit 1,4 bis 2,8 Mol, vorzugsweise 1,6 bis 2,6 Mol, einer gesättigten geradkettigen Monocarbonsäure mit 6 bis 10 C-Atomen oder deren Gemischen und 0,1 bis 0,8 Mol, vorzugsweise 0,2 bis 0,7 Mol einer gesättigten aliphatischen Dicarbonsäure, deren Gemischen oder Anhydriden, wobei die Estergemische Hydroxylzahlen von 5 bis 20 und eine Säurezahl unter 5 besitzen.

2. Verfahren zur Herstellung biologisch vollständig abbaubarer, flüssiger, einheitlicher Estergemische nach Anspruch 1, dadurch gekennzeichnet, daß man je Mol Glycerin mit 1,4 bis 2,8 Mol, vorzugsweise 1,6 bis 2,6 Mol einer gesättigten, geradkettigen Monocarbonsäure mit 6 bis 10 C-Atomen oder deren Gemischen und 0,1 bis 0,8 Mol, vorzugsweise 0,2 bis 0,6 Mol, gesättigten, aliphatischen Dicarbonsäuren, deren Gemischen oder Anhydriden bei 200 bis 250 °C, vorzugsweise unter Vakuum, bei Molverhältnissen von Glycerin zu Dicarbonsäuren unter 1 : 0,6 gemeinsam verestert oder nach Bildung hydroxylhaltiger Partialester der Monocarbonsäure, bis Säurezahlen unter 50, vorzugsweise unter 10, erreicht sind, diese mit 0,1 bis 0,8 Mol Dicarbonsäure verestert, bis eine Hydroxylzahl von 5 bis 20 und eine Säurezahl unter 5, vorzugsweise unter 1, erreicht ist, und daß man das Rohprodukt in üblicher Weise entfärbt und desodorisiert.

3. Verfahren nach Anspruch 2, dadurch gekennzeichnet, daß man Glycerin mit einer gesättigten, geradkettigen Monocarbonsäure mit 6 bis 10 C-Atomen oder deren Gemischen und aliphatischen Dicarbonsäuren, deren Gemischen oder Anhydriden gleichzeitig bei 200-250 °C, vorzugsweise bei 200-240 °C, unter Vakuum verestert.

4. Verfahren nach Anspruch 2, dadurch gekennzeichnet, daß man die hydroxylhaltigen Glycerin-Partialester in Abwesenheit der aliphatischen Dicarbonsäuren, deren Gemischen oder Anhydriden bei 200-250 °C, herstellt, auf 140 °C abkühlt und anschließend daran mit aliphatischen Dicarbonsäuren verestert.

5. Verfahren nach einem der Ansprüche 2 bis 4, dadurch gekennzeichnet, daß das Vakuum der Reaktionsmischung in Anwesenheit der aliphatischen Dicarbonsäuren, deren Gemischen oder Anhydriden langsam um etwa 5-50 mbar, vorzugsweise 10-20 mbar, je Stunde erhöht wird.

## Claims

1. Fully biodegradable, fluid, homogeneous ester mixtures with low cloud points and with viscosities, which are adjustable for particular products, consisting of esterification products of 1 mol of glycerin and 1.4 to 2.8 mol, preferably 1.6 to 2.6 mol, of a saturated straight-chain monocarboxylic acid with 6 to 10 C atoms or mixtures thereof and 0.1 to 0.8 mol, preferably 0.2 to 0.7 mol, of a saturated aliphatic dicarboxylic acid, mixtures or anhydrides thereof, the ester mixtures having hydroxyl numbers of 5 to 20 and an acid number below 5.

2. Process for the preparation of fully biodegradable, fluid, homogeneous ester mixtures, according to claim 1, characterised in that each mol of glycerin is esterified with 1.4 to 2.8 mol, preferably 1.6 to 2.6 mol, of a saturated straight-chain monocarboxylic acid with 6 to 10 C atoms or mixtures thereof and 0.1 to 0.8 mol, preferably 0.2 to 0.6 mol, of saturated aliphatic dicarboxylic acids, mixtures or anhydrides thereof at 200 to 250 °C, preferably in a vacuum, jointly by common esterification of all compounds in case of molar ratios of glycerin to dicarboxylic acids below 1 : 0.6, or after formation of hydroxyl-containing partial esters of the monocarboxylic acid until acid numbers below 50, preferably below 10 are reached, these partial esters are esterified with 0.1 to 0.8 mol of dicarboxylic acid, until a hydroxyl number of 5 to 20 and an acid number below 5, preferably below 1, is reached, and in that the crude product is decolourized and deodorized in the usual way.

3. Process according to claim 2, characterized in that glycerin is esterified in a vacuum at 200 to 250 °C, preferably at 200 to 240 °C, simultaneously with a saturated straight-chain monocarboxylic acid having 6 to 10 carbon atoms or their mixtures and aliphatic dicarboxylic acids, their mixtures or anhydrides.

4. Process according to claim 2, characterized in that the hydroxyl containing glycerin partial esters are formed in the absence of the aliphatic dicarboxylic acids, their mixtures or anhydrides at 200 to 250 °C, there is cooled to 140 °C and these partial esters subsequently are esterified with aliphatic dicarboxylic acids.

5. Process according to one of claims 2 to 4, characterized in that the vacuum of the reaction mixture in the presence of the aliphatic dicarboxylic acids, their mixtures or anhydrides slowly is increased by about 5 to 50 mbar per hour, preferably 10 to 20 mbar per hour.

## Revendications

1. Mélanges d'esters homogènes, liquides, entièrement biodégradables, à faibles points de trouble et de viscosités pouvant être ajustées selon les produits, se composant de produits d'estérification de 1 mol de glycérine avec 1,4 à 2,8 mol, de préférence 1,6 à 2,6 mol, d'un acide monocarboxylique saturé à chaîne droite à 6 à 10 atomes de carbone ou ses mélanges, et 0,1 à 0,8 mol, de préférence 0,2 à 0,7 mol, d'un acide dicarboxylique aliphatique saturé, ses mélanges ou anhydrides, les mélanges d'esters présentant des indices d'hydroxyle de 5 à 20 et un indice d'acide inférieur à 5.

2. Procédé pour la préparation de mélanges d'esters homogènes, liquides, entièrement biodégradables, selon la revendication 1, caractérisé en ce que l'on estérifie chaque mol de glycérine avec 1,4 à 2,8 mol, de préférence 1,6 à 2,6 mol, d'un acide monocarboxylique saturé à chaîne droite à 6 à 10 atomes de carbone ou ses mélanges, et 0,1 à 0,8 mol, de préférence 0,2 à 0,6 mol d'acides dicarboxyliques aliphatiques saturés, leurs mélanges ou anhydrides, à 200 à 250 °C, de préférence sous vide par estérification simultanée de tous les composants en cas de rapports molaires de la glycérine aux acides dicarboxyliques inférieurs à 1 : 0,6, ou après formation d'un ester partiel hydroxylé de l'acide monocarboxylique, jusqu'à obtention d'indices d'acide inférieurs à 50, de préférence inférieurs à 10, avec 0,1 à 0,8 mol d'acide dicarboxylique, jusqu'à ce que l'on obtienne un indice d'hydroxyle de 5 à 20 et un indice d'acide inférieur à 5, de préférence inférieur à 1, et qu'on déclore et désodorise le produit brut de la manière habituelle.

3. Procédé selon la revendication 2, caractérisé en ce qu'on estérifie de la glycérine sous vide, à 200-250 °C, de préférence à 200-240 °C, simultanément avec un acide monocarboxylique saturé à chaîne droite à 6 à 10 atomes de carbone ou ses mélanges et des acides dicarboxyliques aliphatiques, leurs mélanges ou anhydrides.

4. Procédé selon la revendication 2, caractérisé en ce qu'on prépare les esters partiels de glycérine hydroxylés en l'absence des acides dicarboxyliques aliphatiques, leurs mélanges ou anhydrides, de préférence à 200-250 °C, refroidis à 140 °C, et qu'on les estérifie ensuite avec des acides dicarboxyliques aliphatiques.

5. Procédé selon l'une des revendications 2 à 4, caractérisé en ce que le vide du mélange réactionnel en présence des acides dicarboxyliques aliphatiques, leurs mélanges ou anhydrides, est lentement élevé d'environ 5-50 mbar par heure (666 6665 Pa/h), de préférence de 10-20 mbar par heure (1333 2666 Pa/h).